# EUROPEAN PATENT APPLICATION

(11) **EP 3 973 979 A1**
(43) Date of publication of application: **30.03.2022**
(21) Application number: 20198488.7
(22) Date of filing: 25.09.2020
(51) Int. Cl.: A61K 38/17, A61K 48/00, A61P 25/08

(54) **A TIGHT JUNCTION (ZONULAE OCCLUDENTES) PROTEIN FOR USE IN TREATING OR PREVENTING EPILEPSY**

(71) Applicant: The Provost, Fellows, Foundation Scholars, and The Other Members of Board, of The College of The Holy and Undivided Trinity of Queen Elizabeth, Dublin 2 (IE)
(72) Inventor: CAMPBELL, Matthew, Dublin, 2 (IE); GREEN, Chris, Dublin, 2 (IE); HUDSON, Natalie, Dublin, 2 (IE); DOHERTY, Colin, Dublin, 2 (IE)
(74) Representative: FRKelly

(57) **Abstract**

The present invention relates to a tight junction (zonulae occludentes) protein, in particular claudin-5, for use in treating or preventing epilepsy. Also disclosed is a vector useful in treating or preventing epilepsy.

## Description

### Field of the Invention

The present invention relates to a tight junction (zonulae occludentes) protein for use in treating or preventing a neurological disorder. Also disclosed are methods of treating or preventing a neurological disorder in a subject, a vector useful in treating or preventing a neurological disorder, and methods of delivering a vector.

### Background to the Invention

Epilepsy is one of the most common neurological disorders, with a prevalence of 1-2% worldwide. Mesial temporal lobe epilepsy (mTLE) is the most common type of epilepsy in humans. mTLE is frequently caused by traumatic brain injury with hippocampal sclerosis, blood-brain-barrier (BBB) dysfunction and accompanying neurological deficits key features of the disease.

Despite the introduction of a plethora of antiepileptic drugs over recent decades, ∼30% of mTLE patients are non-responsive to medication. As such, surgical resection remains the only viable option for many patients to date. Following surgery, studies have estimated that 53-84% of individuals remain seizure-free for one year but, in certain subgroups, as many as 50% of patients do not remain seizure-free for at least one year after removal of the epileptic foci. Therefore, it is imperative to identify and develop novel therapeutics for the treatment of neurological disorders such as epilepsy, in particular drug-resistant TLE.

The BBB is formed by brain microvascular endothelial cells, astrocytes, pericytes and microglia collectively maintaining key properties to strictly regulate the movement of material between the blood and the brain. This strict regulation of molecular exchange is enforced by the presence of tight junction complexes sealing the space between endothelial cells (paracellular) and by the presence of luminal and abluminal receptors controlling the passage of molecules across cells (transcellular).

It has been known since the 1990s that defects in the BBB can result in seizures and epilepsy. For example, mutations to Glut-1, the glucose transporter enriched on brain endothelial cells, leads to infantile seizures due to impaired glucose transport to the brain. One of the largest risk factors for the development of epilepsy, traumatic brain injury, is intimately linked to BBB disruption. Indeed, where causes could be identified, traumatic brain injury accounted for 30% of cases of epilepsy in 15-34 year olds. Furthermore, it has been shown that in non-epileptic animals, focal disruption of the BBB can lead to seizures via cortical application of mannitol. The degree of BBB disruption also correlates with seizure severity. In epilepsy, loss of BBB integrity occurs early in the progression of the disease and resulting BBB dysfunction primes the brain for immune cell recruitment and subsequent neuronal damage. Additionally, the increased permeability of serum-derived molecules including albumin, fibrinogen and immunoglobulin can lead to pro-inflammatory responses due to the activation of surrounding cell types such as astrocytes and microglia. For example, albumin extravasated from the serum to the brain parenchyma can activate TGFβ-1 signalling and potentiate epileptic seizures and BBB disruption, in part due to disruption of tight junction complexes.

Tight junction proteins regulate paracellular permeability between endothelial cells. Numerous tight junction proteins including claudins, occludin, LSR and tricellulin have been described as playing important roles in maintaining BBB integrity. Of these proteins, claudin-5 is the most abundant and most commonly linked to neurological disease. Claudin-5 is a member of the claudin multigene family which consists of up to 27 members. It forms reciprocal interactions with junctional proteins on adjacent endothelial cells to effectively seal the paracellular space. It is expressed in many organs but is particularly enriched in brain endothelial cells where it is vital for the function of the BBB. Claudin-5 is the most enriched tight junction protein in brain endothelial cells and claudin-5 knockout mice die soon after birth and have a disrupted BBB to low molecular weight molecules.

There have been significant advances in the field of gene therapy in recent years. Adeno-associated virus (AAV) is an attractive delivery vector due to its low immunogenicity and ability to transduce non-dividing cells. AAV can infect a variety of cell and tissue types and is a promising approach to treat human disorders. AAV technology was recently approved by the FDA for use in a rare form of blindness, Leber Congenital Amaoursis (LCA). Additionally, there are currently 7 studies using AAV9 actively recruiting in the USA. However, at present there have been no clinical trials assessing gene therapies for the treatment of epilepsy. The current paradigm in the treatment of CNS disorders has been to identify delivery vehicles capable of bypassing the BBB or developing methods to overcome it. As such, there has been little research on strategies to restore BBB function.

To date, no gene therapies have been developed to specifically modulate and regulate BBB components.

### Summary of the Invention

According to a first aspect of the present invention, there is provided a tight junction (zonulae occludentes) protein for use in treating or preventing a neurological disorder in a subject.

Optionally, the use comprises administering a tight junction protein to the subject.

According to a second aspect of the present invention, there is provided use of a tight junction protein in the manufacture of a medicament for treating or preventing a neurological disorder.

According to a third aspect of the present invention, there is provided a method of treating or preventing a neurological disorder in a subject, the method comprising administering a tight junction protein to the subject.

Optionally, the neurological disorder is selected from epilepsy, schizophrenia, multiple sclerosis, stroke, and traumatic brain injury.

Preferably, the neurological disorder is epilepsy. Further preferably, the neurological disorder is temporal lobe epilepsy. Still further preferably, the neurological disorder is mesial temporal lobe epilepsy.

Optionally, the neurological disorder is drug-resistant epilepsy. Further preferably, the neurological disorder is drug-resistant temporal lobe epilepsy. Still further preferably, the neurological disorder is drug-resistant mesial temporal lobe epilepsy.

Optionally, the use comprises treating or preventing epilepsy. Further optionally, the use comprises treating or preventing epileptic seizures. Still further optionally, the use comprises treating or preventing convulsive epileptic seizures. Still further optionally, the use comprises treating or preventing focal epileptic seizures.

Optionally, the medicament is for treating or preventing epilepsy. Further optionally, the medicament is for treating or preventing epileptic seizures. Still further optionally, the medicament is for treating or preventing convulsive epileptic seizures. Still further optionally, the medicament is for treating or preventing focal epileptic seizures.

Optionally, the method is a method of treating or preventing epilepsy. Further optionally, the method is a method of treating or preventing epileptic seizures. Still further optionally, the method is a method of treating or preventing convulsive epileptic seizures. Still further optionally, the method is a method of treating or preventing focal epileptic seizures.

Optionally, the tight junction protein is a claudin protein.

Preferably, the tight junction protein is a claudin-5 protein.

Optionally, the tight junction protein is a mouse claudin-5 protein. Alternatively, the tight junction protein is a human claudin-5 protein.

Preferably, the tight junction protein is a human claudin-5 protein.

Optionally, the use or method comprises maintaining the quantitative or qualitative level of the tight junction protein. Further optionally, the use or method comprises increasing the quantitative or qualitative level of the tight junction protein. Still further optionally, the use or method comprises increasing the quantitative or qualitative level of the tight junction protein in the brain. Still further optionally, the use or method comprises increasing the quantitative or qualitative level of the tight junction protein in an endothelial cell of the brain.

Optionally, the use or method comprises maintaining the quantitative or qualitative level of the claudin protein. Further optionally, the use or method comprises increasing the quantitative or qualitative level of the claudin protein. Still further optionally, the use or method comprises increasing the quantitative or qualitative level of the claudin protein in the brain. Still further optionally, the use or method comprises increasing the quantitative or qualitative level of the claudin protein in an endothelial cell of the brain.

Optionally, the use or method comprises maintaining the quantitative or qualitative level of the claudin-5 protein. Further optionally, the use or method comprises increasing the quantitative or qualitative level of the claudin-5 protein. Still further optionally, the use or method comprises increasing the quantitative or qualitative level of the claudin-5 protein in the brain. Still further optionally, the use or method comprises increasing the quantitative or qualitative level of the claudin-5 protein in an endothelial cell of the brain.

Optionally, the use or method comprises administering the tight junction protein to the subject and maintaining the quantitative or qualitative level of the tight junction protein. Further optionally, the use or method comprises administering the tight junction protein to the subject and increasing the quantitative or qualitative level of the tight junction protein. Still further optionally, the use or method comprises administering the tight junction protein to the subject and increasing the quantitative or qualitative level of the tight junction protein in the brain. Still further optionally, the use or method comprises administering the tight junction protein to the subject and increasing the quantitative or qualitative level of the tight junction protein in in an endothelial cell of the brain.

Optionally, the use or method comprises administering an agent capable of maintaining the quantitative or qualitative level of the tight junction protein to the subject. Further optionally, the use or method comprises administering an agent capable of increasing the quantitative or qualitative level of the tight junction protein to the subject. Still further optionally, the use or method comprises administering an agent capable of increasing the quantitative or qualitative level of the tight junction protein in the brain to the subject. Still further optionally, the use or method comprises administering an agent capable of increasing the quantitative or qualitative level of the tight junction protein in in an endothelial cell of the brain to the subject.

Optionally, the use or method comprises administering an agent capable of maintaining expression of the tight junction protein in the subject. Further optionally, the use or method comprises administering an agent capable of increasing expression of the tight junction protein in the subject. Still further optionally, the use or method comprises administering an agent capable of increasing expression of the tight junction protein in the brain in the subject. Still further optionally, the use or method comprises administering an agent capable of increasing expression of the tight junction protein in in an endothelial cell of the brain in the subject.

Optionally, the agent is a peptide or protein. Further optionally, the agent is a peptide or protein selected from glial cell line-derived neurotrophic factor (GDNF), adrenomedullin, SOX-18

Optionally, the agent is a compound. Further optionally, the agent is a steroid compound. Still further optionally, the agent is a corticosteroid compound.

Optionally, the agent is a glucocorticoid compound.

Optionally, the agent is a gonadocorticoid compound. Further optionally, the agent is estrogen.

Optionally, the agent is a nucleic acid encoding a tight junction protein, or is fragment or variant thereof. Further optionally, the agent is an isolated nucleic acid encoding a tight junction protein or is a fragment or variant thereof. Still further optionally, the agent is an isolated nucleic acid encoding a tight junction protein operatively linked to a nucleic acid comprising a promoter, or is fragment or variant thereof.

Optionally, the agent is a nucleic acid encoding a claudin protein, or is fragment or variant thereof. Further optionally, the agent is an isolated nucleic acid encoding a claudin protein or is a fragment or variant thereof. Still further optionally, the agent is an isolated nucleic acid encoding a claudin protein operatively linked to a nucleic acid comprising a promoter, or is fragment or variant thereof.

Optionally, the agent is a nucleic acid encoding a claudin-5 protein, or is fragment or variant thereof. Further optionally, the agent is an isolated nucleic acid encoding a claudin-5 protein or is a fragment or variant thereof. Still further optionally, the agent is an isolated nucleic acid encoding a claudin-5 protein operatively linked to a nucleic acid comprising a promoter, or is fragment or variant thereof.

Optionally, the agent is a vector comprising a nucleic acid encoding a tight junction protein, or fragment or variant thereof, operatively linked to a nucleic acid comprising a promoter, or fragment or variant thereof.

Optionally, the agent is a vector comprising a nucleic acid encoding a claudin protein, or fragment or variant thereof, operatively linked to a nucleic acid comprising a promoter, or fragment or variant thereof. Further optionally, the agent is a vector comprising a nucleic acid encoding a human claudin protein, or fragment or variant thereof, operatively linked to a nucleic acid comprising a promoter, or fragment or variant thereof.

Optionally, the agent is a vector comprising a nucleic acid encoding a claudin-5 protein, or fragment or variant thereof, operatively linked to a nucleic acid comprising a promoter, or fragment or variant thereof. Further optionally, the agent is a vector comprising a nucleic acid encoding a human claudin-5 protein, or fragment or variant thereof, operatively linked to a nucleic acid comprising a promoter, or fragment or variant thereof.

According to a fourth aspect of the present invention, there is provided a vector comprising a first nucleic acid encoding a tight junction protein, or fragment or variant thereof, operatively linked to a second nucleic acid comprising a promoter, or fragment or variant thereof.

Optionally, the first nucleic acid or fragment or variant thereof encodes a claudin protein. Further optionally, the first nucleic acid or fragment or variant thereof encodes a human claudin protein.

Optionally, the first nucleic acid or fragment or variant thereof encodes a claudin-5 protein. Further optionally, the first nucleic acid or fragment or variant thereof encodes a human claudin-5 protein.

Optionally, the second nucleic acid comprises a cell-type-specific promoter. Further optionally, the second nucleic acid comprises an endothelial-cell-specific promoter.

Optionally, the second nucleic acid comprises a claudin-5 promoter or fragment or variant thereof. Further optionally, the second nucleic acid comprises a human claudin-5 promoter or fragment or variant thereof.

Alternatively, the second nucleic acid comprises an intercellular adhesion molecule (ICAM) promoter or fragment or variant thereof. Further optionally, the second nucleic acid comprises a human intercellular adhesion molecule (ICAM) promoter or fragment or variant thereof.

Further alternatively, the second nucleic acid comprises an intercellular adhesion molecule 2 (ICAM2) promoter or fragment or variant thereof. Further optionally, the second nucleic acid comprises a human intercellular adhesion molecule 2 (ICAM2) promoter or fragment or variant thereof.

Optionally, the variant is a nucleic acid having a nucleic acid sequence with at least 70% sequence identity to the first or second nucleic acid. Further optionally, the variant is a nucleic acid having a nucleic acid sequence with at least 75%, optionally at least 80%, optionally at least 85%, optionally at least 90%, optionally at least 95%, optionally at least 96%, optionally at least 97%, optionally at least 98%, optionally at least 99% sequence identity to the first or second nucleic acid.

Optionally, the first nucleic acid has a nucleic acid sequence according to any one or more of SEQ ID NO:1, SEQ ID NO:2, SEQ ID NO:3, and SEQ ID NO:4.

Further optionally, the first nucleic acid has a nucleic acid sequence according to any one or more of SEQ ID NO:1, and SEQ ID NO:2.

Preferably, the first nucleic acid has a nucleic acid sequence according to SEQ ID NO:2.

Optionally, the second nucleic acid has a nucleic acid sequence according to any one or more of SEQ ID NO:5, and SEQ ID NO:6.

Preferably, the second nucleic acid has a nucleic acid sequence according to SEQ ID NO:6.

Preferably, the vector comprises a first nucleic acid having a nucleic acid sequence according to SEQ ID NO:2, or fragment or variant thereof, operatively linked to a second nucleic acid having a nucleic acid sequence according to SEQ ID NO:6, or fragment or variant thereof.

**Table 1. Nucleic acid sequences**

| **SEQ ID NO:** | **Nucleic acid sequence name** | **Nucleic acid sequence** |
|---|---|---|
| 1 | Human Claudin-5 cDNA | ATGACCCGCGCACGGATTGGCTGCTTCGGGCCGGGG GGCCGGGCCCGGGGACAGAATCCGCCCCCGAACCT TCAAAGAGGGTACCCCCGGCAGGAGCTGGCAGACCC AGGAGGTGCGACAGACCCGCGGGCAAACGGACTGG GGCCAAGAGCCGGGAGCGCGGGCGCAAAGGCACCAG GGCCCGCCCAGGGCGCCGCGCAGCACGGCCTTGGGG GTTCTGCGGGCCTTCGGGTGCGCGTCTCGCCTCTAGC CATGGGGTCCGCAGCGTTGGAGATCCTGGGCCTGGTG CTGTGCCTGGTGGGCTGGGGGGTCTGATCCTGGCGT GCGGGCTGCCCATGTGGCAGGTGACCGCCTTCCTGGA CCACAACATCGTGACGGCGCAGACCACCTGGAAGGGG CTGTGGATGTCGTGCGTGGTGCAGAGCACCGGGCACA TGCAGTGCAAAGTGTACGACTCGGTGCTGGCTCTGAG CACCGAGGTGCAGGCGGCGCGGGCGCTCACCGTGAG CGCCGTGCTGCTGGCGTTCGTTGCGCTGTTCGTGACC CTGGCGGGCGCGCAGTGCACCACCTGCGTGGCCCCG GGCCCGGCCAAGGCGCGTGTGGCCCTCACGGGAGGC GTGCTCTACCTGTTTTGCGGGCTGCTGGCGCTCGTGC CACTCTGCTGGTTCGCCAACATTGTCGTCCGCGAGTTT TACGACCCGTCTGTGCCCGTGTCGCAGAAGTACGAGC TGGGCGCAGCGCTGTACATCGGCTGGGCGGCCACCG CGCTGCTCATGGTAGGCGGCTGCCTCTTGTGCTGCGG CGCCTGGGTCTGCACCGGCCGTCCCGACCTCAGCTTC |
| | | CCCGTGAAGTACTCAGCGCCGCGGCGGCCCACGGCC ACCGGCGACTACGACAAGAAGAACTACGTCTGA |
| 2 | Human Claudin-5 cDNA codon-optimised | ATGACTAGGGCCCGGATTGGATGCTTCGGTCCCGGCG GACGCGCGCGCGGAACCGAGTCAGCCCCCGAACCGA GCAAACGCGTGCCACCCGGAAGATCATGGCAGACCCA AGAAGTCAGACAGACCCGGGGCGCCAACGGCTGGG TCCTCGGGCCGGATCCGCGGGGGCCAAGGCACCTGG GCCAGCTCAGGGTGCCGCACAGCACGGCCTGGGCGG AAGCGCAGGGTTGAGGGTCCGGGTGTCCCCTCTGGCC ATGGGATCGGCCGCCCTCGAGATTTTGGGCCTGGTGC TGTGCCTGGTCGGATGGGGCGGACTGATCCTGGCCTG CGGACTCCCGATGTGGCAAGTCACCGCCTTTCTCGAT CATAACATCGTGACGGCCCAGACCACCTGGAAGGGCC TGTGGATGTCGTGCGTGGTGCAGTCCACTGGCCACAT GCAGTGCAAGGTCTACGACTCCGTGCTGGCGCTGTCC ACTGAGGTGCAGGCCGCCCGGGCCCTCACTGTGTCCG CCGTGCTTCTCGCTTTCGTGGCGCTGTTCGTGACCCTG GCCGGTGCTCAGTGCACTACCTGTGTGGCGCCGGGCC CCGCCAAGGCCCGCGTGGCCCTGACTGGGGGAGTGC TGTACCTCTTCTGTGGCCTCCTTGCCCTCGTGCCGCTG TGCTGGTTCGCGAATATCGTGGTCCGGGAATTCTATGA CCCTTCGGTGCCGGTGTCCCAAAAATACGAACTTGGTG CTGCACTGTACATCGGATGGGCCGCAACAGCGCTGCT GATGGTCGGCGGCTGCCTGCTGTGCTGCGGAGCCTG GGTCTGTACCGGACGCCCCGACTTGAGCTTCCCCGTG AAGTACAGCGCCCCTAGACGGCCGACCGCTACCGGG GATTACGACAAGAAGAACTACGTGTGA |
| 3 | Mouse Claudin-5 cDNA | ATGGGGTCTGCAGCGTTGGAAATTCTGGGTCTGGTGC TGTGTCTGGTAGGATGGGTGGGCTTGATCCTGGCGTG TGGGCTGCCCATGTGGCAGGTGACTGCCTTCCTGGAC CACAACATCGTGACGGCGCAGACGACTTGGAAGGGGC TGTGGATGTCGTGCGTGGTGCAGAGTACCGGGCACAT GCAGTGCAAGGTGTATGAATCTGTGCTGGCGCTGAGT GCGGAGGTGCAGGCAGCTCGGGCACTCACCGTGGGC GCTGTGCTGCTGGCGCTGGTGGCACTCTTTGTTACCTT GACCGGCGCTCAGTGCACCACCTGCGTGGCCCCGGG CCCAGTTAAGGCACGGGTAGCACTCACGGGAGGAGCG CTTTACGCGGTGTGCGGGCTGCTGGCACTCGTGCCGC TCTGCTGGTTCGCCAACATCGTTGTCCGCGAGTTCTAT GATCCGACGGTGCCGGTGTCACAGAAGTACGAGCTGG |
| | | GCGCGGCGCTGTACATCGGCTGGGCGGCCTCCGCAC TGCTCATGTGCGGTGGCGGCCTCGTGTGTTGCGGCGC CTGGGTCTGCACCGGGCGCCCTGAGTTCAGCTTCCCG GTCAAGTACTCTGCGCCGCGGCGGCCCACGGCCAATG GCGATTACGACAAGAAGAACTATGTCTAA |
| 4 | Mouse Claudin-5 cDNA codon-optimised | ATGGGAAGCGCCGCATTGGAAATCCTCGGGCTGGTGC TGTGCCTCGTGGGATGGGTCGGACTGATTCTGGCTTG CGGACTGCCGATGTGGCAAGTCACCGCCTTCCTGGAC CACAACATCGTGACTGCGCAGACCACTTGGAAGGGTC TGTGGATGTCGTGTGTGGTCCAGTCCACCGGCCATAT GCAGTGCAAAGTCTACGAGTCCGTGCTCGCCTTGTCC GCCGAGGTGCAAGCTGCGAGAGCCCTGACCGTGGGG GCCGTGCTGCTGGCCCTGGTGGCGCTTTTCGTGACAC TGACGGGTGCCCAGTGTACTACCTGTGTGGCCCCGGG CCCCGTGAAGGCCCGGGTGGCCCTCACTGGCGGAGC CCTGTACGCCGTGTGCGGGCTGCTGGCGCTGGTGCC CCTGTGCTGGTTCGCCAACATCGTCGTGCGCGAGTTC TACGATCCTACCGTGCCCGTCAGCCAGAAATACGAACT TGGAGCCGCTCTGTATATTGGCTGGGCTGCATCGGCC CTCCTTATGTGCGGTGGCGGCCTCGTGTGCTGCGGGG CATGGGTCTGCACCGGACGCCCGGAATTCTCCTTTCCT GTGAAGTACTCAGCCCCACGGAGGCCTACCGCAACG GAGACTACGACAAGAAGAATTACGTGTGA |
| 5 | Claudin-5 promoter | AACAAAGGGAGCAGGGAGGAGTGTTCACTGGAAAGAG GCTGGCGCCCTAGTTTGGTTGTGGGCTGTGGGGCCTG GTGCCCCCACCTGAGCCTCAGGGACCCAGTGTGTCTA ACCCAGTGGACCTTTCAAGAAATGGCTGGGCCATTGTG CAGAAGAATGCCCGGAAATCCCGCGGCTCCCTCCTCC ACCGAGGATGGGGGCTCTTGCTCCTGGCCAGGAAACT CCAAGTTGGCTTCCGGAGGGTGGCCTGGGGGCTGGG GTGGCAAAGCACCACAACTATTTGTAGGATGGCAAGG CATAGCCTTTCTATGTCCCATCGTGTCTCTGCTGTCCC AGGATTCCAGTATGCTGTGTTAGGATCACAGCCTATCT GCTGGAGATGACTGGACGTCGGAGAACCCTCAGGGGT TGCGCAGCATCCCAGAGGAGAGCACAGGCCCTTGCAG TGTTCACTTTGTGTCAGACCAGTGGTTCCAGGGAAGGA GTGAGAACTGTAGGCGGTGGTTGGGTAGGATCATGAA TGAGGTTATAATTAAGGGCAAGCCCAACACAGGCTTGC CGAGCATCTCCGCTTGGTGTGCCAAGGGAAAAACGGC GGGCGTAGAGGTTTGTTTCACCTGTGCACGGGGGCTC AGAGAACCTCCAGGCCAAGCAGTCTGCAGAGAGGAGT |
| | | TGGAATGATCCGGGATGTAGCAGGTGGAGCTGGTGAG TGTAGGTTAGGGGTCATGCTGCTAACAGTGGAGACAG GGGCGCACAGATCTCCTGTGTACCTGCCAAGATCAGC CCCTACTAGGACAGAAACTGGTAGGGACTCAGACTGC GGTTCTGCAGGGTGCGGTTCGGGTGAGCAGGACTAGT CCTGCTCGCCCGTGGTTTCAGTAACTGTAGCTGTCAGG GGGTCCGCGATTGGCTGTGGGCCGAAGGTCTGCTCAT GGATTGGTTGAGCTAACTCGGGGGGCTGCGCCCTGGG GGCAGAGTCCGCCCCCGAGACTTCAAATAGGACAACA GGTTACTTCAGAACCCAGTTGGTGTAGTTAAAACCTCC TCTTCTGCTCCAGGACTGGAGGCTCCAGAGCAGAGGC ACCAGAATCAATTCCCAGCTCCCAGCCTAAGCAGCGCA GAGAGCACCCGGAGGCCCCAAGGGCCGTCGGGTGAG CATTCAGTCTTTAGCC |
| 6 | ICAM2 promoter | ATGGGATTTGGGGTTCCCCAGATCTGGGGCTTGTAGG CCTGACTCTCCCCTGTGCACACGTCTCATACACGCATG CGTGCACCCATTGCCTGCCCCGCCCCTTGCACAGGGA GTCAGCAGGGAGGACTGGGTTATGCCCTGCTTATCAG CAGCTTCCCAGCTTCCTCTGCCTGGATTCTTAGAGGCC TGGGGTCCTAGAACGAGCTGGTGCACGTGGCTTCCCA AAGATCTCTCAGATAATGAGAGGAAATGCAGTCATCAG TTTGCAGAAGGCTAGGGATTCTGGGCCATAGCTCAGA CCTGCGCCCACCATCTCCCTCCAGGCAGCCCTTGGCT GGTCCCTGCGAGCCCGTGGAGACTGCCAGAG |

According to a fifth aspect of the present invention, there is provided a virus comprising a vector according to the fourth aspect of the present invention.

Optionally, the virus is an adeno-associated virus (AAV) comprising the vector. Alternatively, the virus is a lentivirus comprising the vector.

Preferably, the virus is an adeno-associated virus. Further preferably, the virus is an isolated adeno-associated virus.

Optionally, the adeno-associated virus is adeno-associated virus serotype 9 (AAV9).

Optionally, the adeno-associated virus comprises an adeno-associated virus capsid and the vector.

Optionally, the adeno-associated virus comprises an adeno-associated virus serotype 9 capsid and the vector.

Optionally, the adeno-associated virus comprises adeno-associated virus inverted terminal repeat sequences and the vector.

Optionally, the adeno-associated virus comprises adeno-associated virus inverted terminal repeat sequences, each adeno-associated virus inverted terminal repeat sequence flanking the vector. Further optionally, the adeno-associated virus comprises adeno-associated virus inverted terminal repeat sequences; each adeno-associated virus inverted terminal repeat sequence flanking opposed respective ends of the vector.

According to a sixth aspect of the present invention, there is provided a composition comprising the adeno-associated virus according to the fifth aspect of the present invention.

Optionally, the composition is a pharmaceutical composition. Further optionally, the composition is a pharmaceutical composition comprising the adeno-associated virus and a pharmaceutically acceptable carrier.

According to a seventh aspect of the present invention, there is provided a host cell transfected with the adeno-associated virus according to the fifth aspect of the present invention or a composition according to the sixth aspect of the present invention.

Optionally, the host cell is an endothelial cell. Further optionally, the host cell is an endothelial cell of the brain.

According to an eighth aspect of the present invention, there is provided a method of delivering a vector according to the fourth aspect of the present invention to a host cell, the method comprising contacting the host cell with the adeno-associated virus according to the fifth aspect of the present invention or a composition according to the sixth aspect of the present invention.

Optionally, the host cell is an endothelial cell. Further optionally, the host cell is an endothelial cell of the brain.

Optionally, the contacting step comprises administering the adeno-associated virus or the composition to the host cell.

Optionally, the administering step comprises parenteral administration.

Optionally, the administering step comprises intravenous, intramuscular, subcutaneous, intradermal, or stereotaxic administration.

According to a ninth aspect of the present invention, there is provided a composition for use in treating or preventing a neurological disorder in a subject, the composition comprising a vector according to a fourth aspect of the present invention, or a virus according to a fifth aspect of the present invention.

Optionally, the use comprises administering a tight junction protein to the subject.

According to a second aspect of the present invention, there is provided use of a vector according to a fourth aspect of the present invention, or a virus according to a fifth aspect of the present invention, in the manufacture of a medicament for treating or preventing a neurological disorder.

According to a third aspect of the present invention, there is provided a method of treating or preventing a neurological disorder in a subject, the method comprising administering a composition comprising a vector according to a fourth aspect of the present invention, or a virus according to a fifth aspect of the present invention.

### Brief Description of the Drawings

Embodiments of the present invention will be described with reference to the appended non-limiting examples and the accompanying drawings in which:

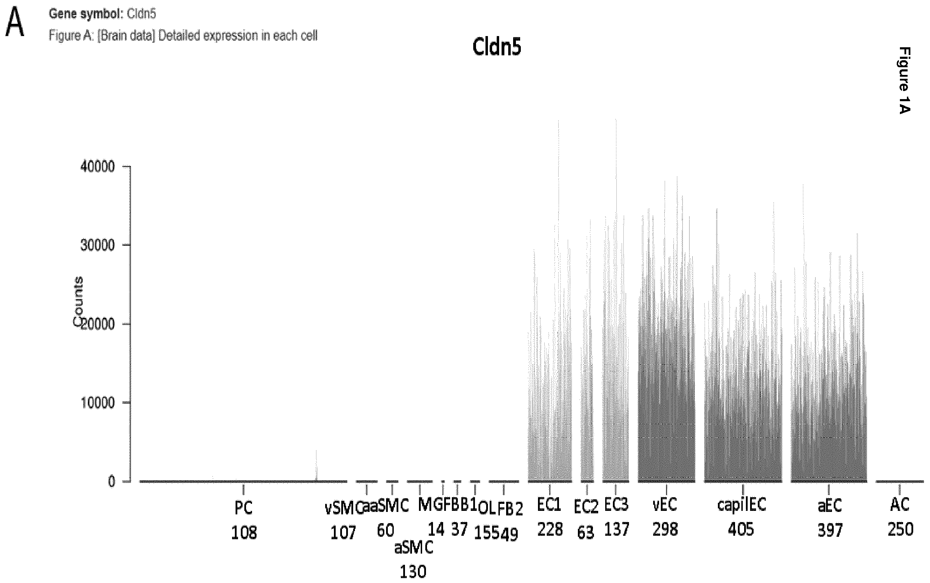

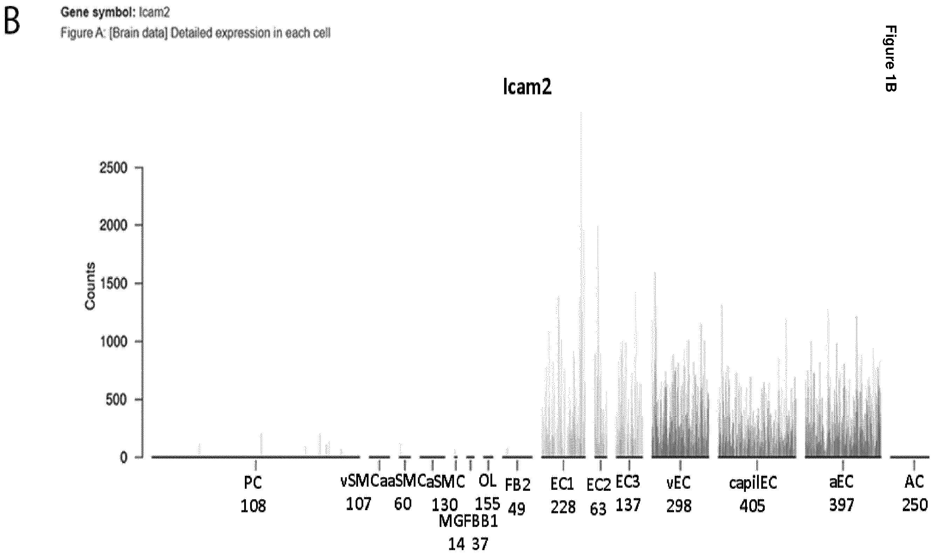

**Figure 1** illustrates detailed mRNA expression profiles of Cldn5 (A) and Icam2 (B) in endothelial cells, pericytes (PC), smooth muscle cells (SMC), fibroblasts (FB), oligodendrocytes (OL) and astrocytes (AC), wherein importantly, Cldn5 and Icam2 are expressed in endothelial cells only;

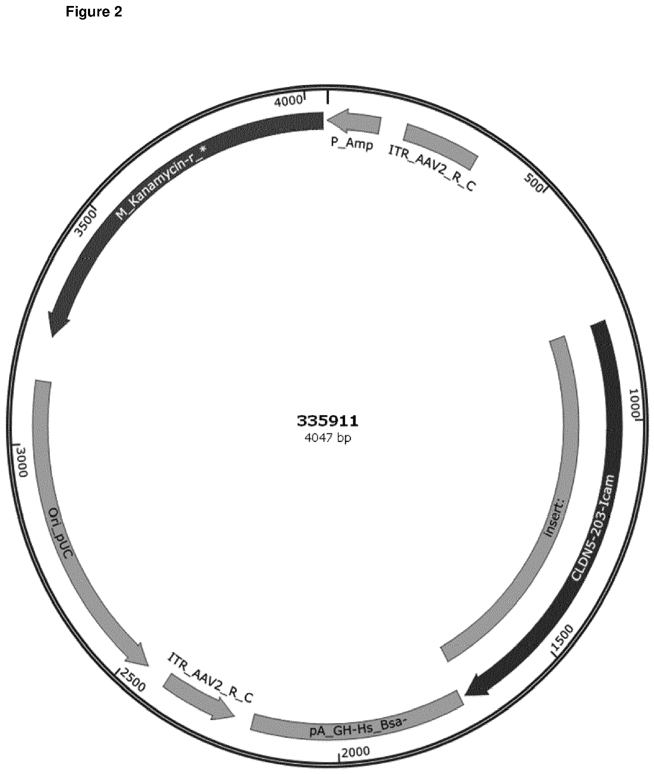

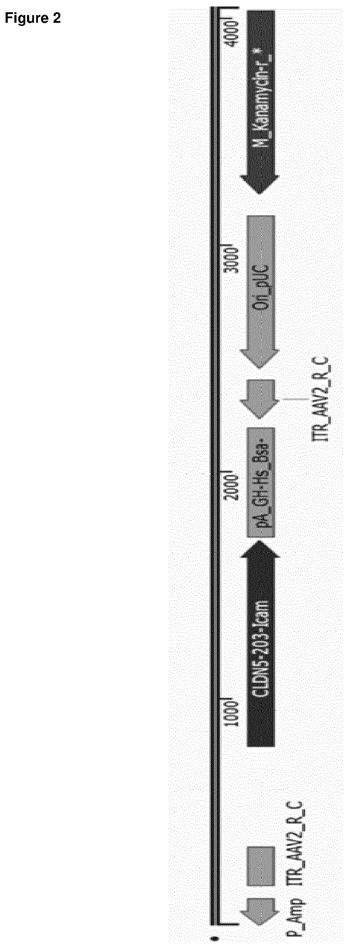

**Figure 2** illustrates vector maps for constitutive expression of Cldn5 under the control of a Cldn5 or Icam2 promoter, wherein for the native vector, claudin-5 expression is constitutively activated by Cldn5 or Icam2 promoters;

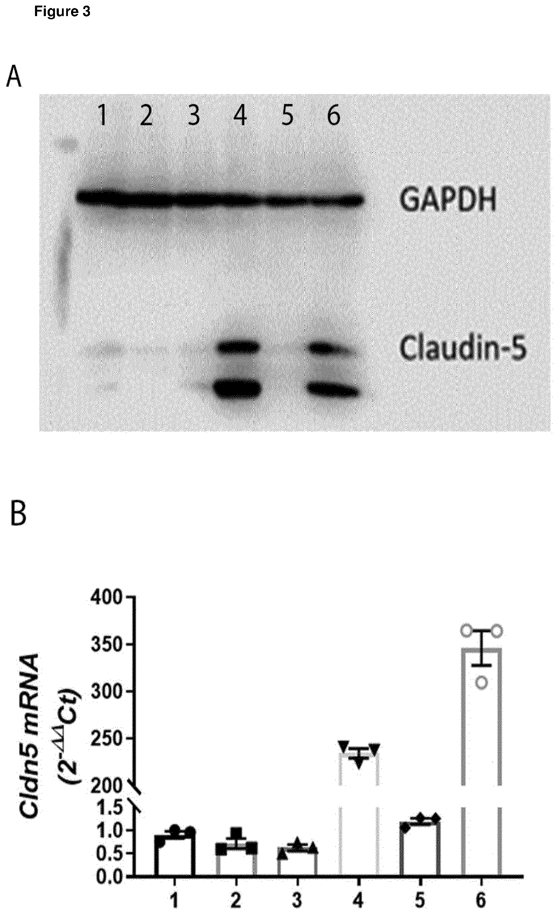

**Figure 3** illustrates a (A) Western blot analysis of claudin-5 and GAPDH in human cerebral microvascular endothelial cells (hCMEC/d3) transfected with claudin-5 expressing plasmids; (B) qPCR analysis of claudin-5 in hCMEC/d3 cells transfected with claudin-5 expressing plasmids, wherein β-actin was used for qPCR normalisation, wherein 1 = untransfected; 2 = empty vector; 3 = claudin-5 promoter and mouse claudin-5 cDNA; 4 = claudin-5 promoter and human claudin-5 cDNA; 5 = ICAM2 promoter and mouse claudin-5 cDNA; 6 = ICAM2 promoter and human claudin-5 cDNA, wherein claudin-5 expression is increased 200-350 fold in cells transfected with claudin-5 plasmids;

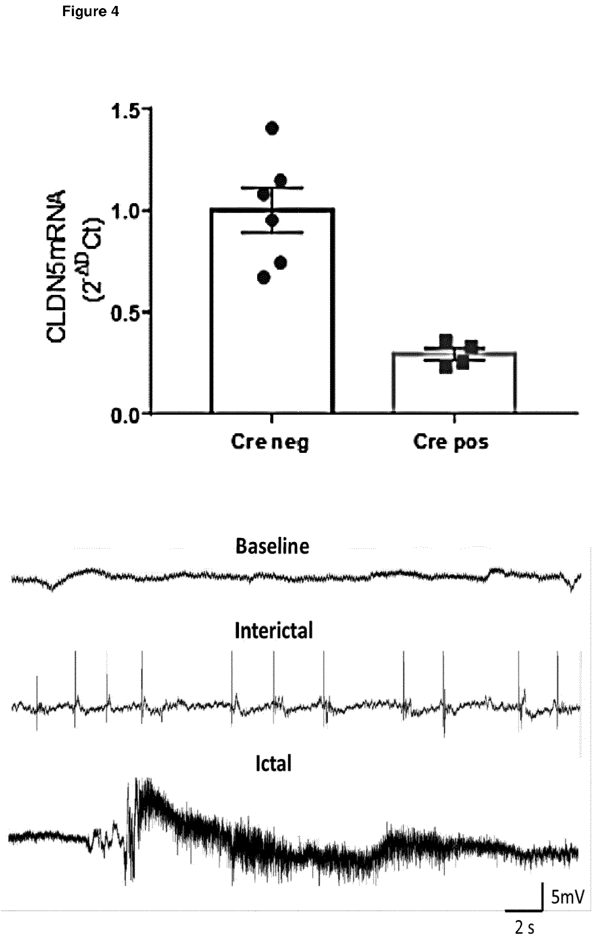

**Figure 4** illustrates (A) Claudin-5 mRNA expression in wild-type (cre neg) and doxycycline-inducible claudin-5 knockdown mice (cre pos), and (B) Claudin-5 deficient mice show epileptiform activity when assessed by cortical electroencephalography;

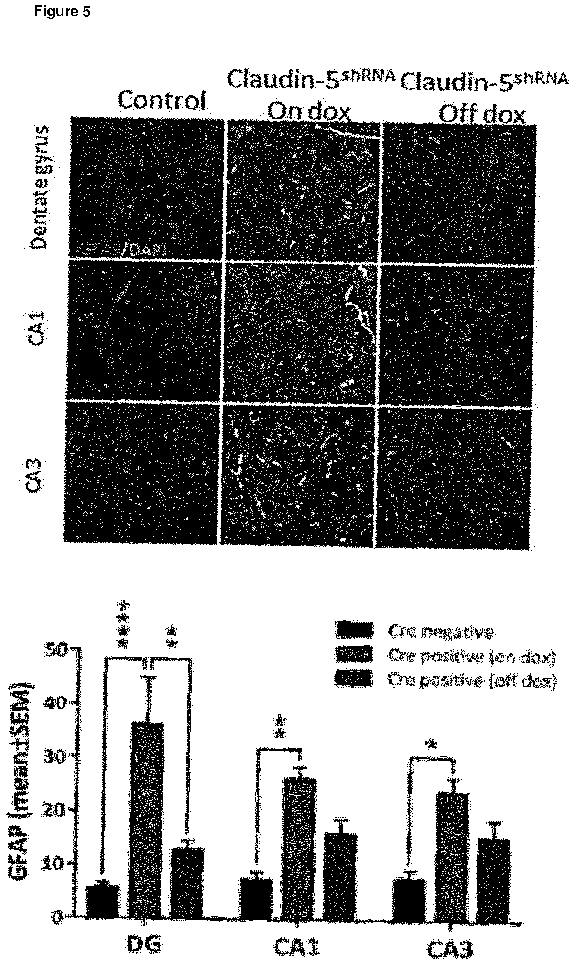

**Figure 5** illustrates (A) immunohistochemistry for GFAP in the hippocampus of control wild-type mice and doxycycline-inducible claudin-5 knockdown mice, wherein mice were supplemented with 2 mg/ml doxycycline in drinking water for 4 weeks and then one group was switched to drinking water only, and (B) quantification of GFAP intensity in the dentate gyrus (DG), CA1 and CA3 region of the Hippocampus, wherein removal of doxycycline returns GFAP expression to wild-type levels; and

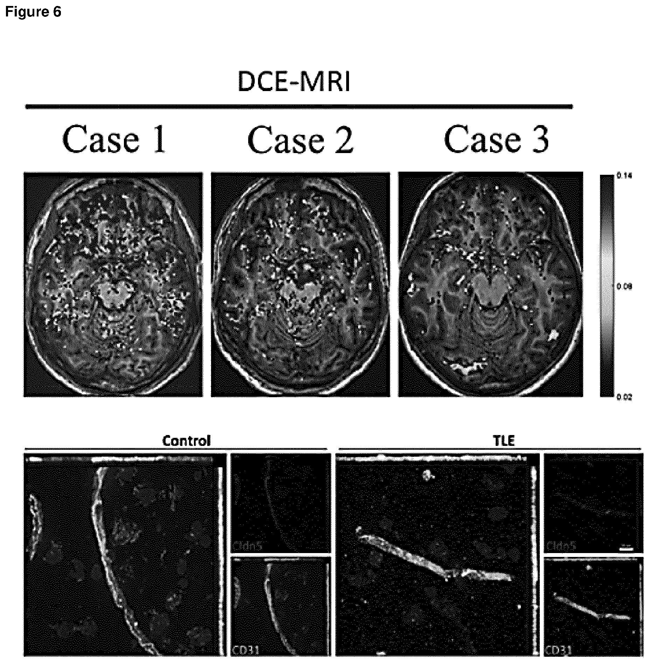

**Figure 6** illustrates (A) blood-brain barrier breakdown in 3 patients with temporal lobe epilepsy as detected by gadolinium (570 Da) leakage during dynamic contrast-enhanced MRI, and (B) immunohistochemistry for claudin-5 and cd31 in autopsy control brain tissue and brain tissue resected from a patient with TLE, demonstrating the loss of continuity of claudin-5 staining in the TLE case.

### Examples

### Materials and Methods

### Animal experiments

All studies were carried out in the Smurfit Institute of Genetics in Trinity College Dublin (TCD) and adhered to the principles laid out by the internal ethics committee at TCD and all relevant national licences were obtained prior to commencement of all studies. All mice were bred on-site in the specific pathogen-free unit at the Smurfit Institute of Genetics in TCD. Mice were housed under a 12-h light/dark cycle at constant temperature and humidity and provided access to standard food and water. Animals were acclimated to their environment for 1 week prior to experimental procedures. Doxycycline-inducible claudin-5 knockdown mice were fed doxycycline (2 mg/ml; 2% sucrose) in their drinking water which was replenished 3 times per week.

### Single cell expression data

Single cell mRNA expression charts were downloaded from the Betsholtz lab single cell RNA sequencing database at http://betsholtzlab.org/VascularSingleCells/database.html as described by Vanlandewijck, M., et al., A molecular atlas of cell types and zonation in the brain vasculature. Nature, 2018. 554(7693): p. 475-480.

### Transfection of CLDN5 expression constructs

The human cerebral microvascular endothelial cells line (hCMEC/d3) was cultured in EGM2-MV media. hCMEC/d3 cells were seeded at 2x10⁵ cells/ml in 12 well plates for 24 hours and transfected with 500 ng of expression constructs using lipofectamine 2000 transfection reagent according to the manufacturer's instructions. 24 hours post-transfection, cells were harvested for RNA and protein analysis.

### Real-time qPCR

RNA was isolated by Trizol extraction and cDNA was reverse transcribed from RNA with the High-Capacity cDNA Reverse Transcription Kit (Applied Biosystems). Transcript levels were quantified on the StepOne Plus instrument (Applied Biosystems) with FastStart Universal SYBR Green Master (ROX) master mix (Roche). The RT-PCR reaction conditions were as follows: 95°C x 2min, (95°C x 5s, 60°C x 30s) x 40, 95°C x 15s, 60°C x 1min, 95°C x 15s, 60°C x 15s. Relative gene expression levels were measured using the comparative *C*_{T} method (ΔΔ*C*_{T}). Expression levels of target genes were normalized to the housekeeping gene β-actin.

### Western blot

10µg of protein lysate was separated on a 10% SDS-PAGE gel and transferred to PVDF membranes at a constant 12V for 2 hours. Membranes were blocked in 5% milk and incubated overnight in primary antibody for claudin-5 (1/1000 diluted in 2% BSA) at 4°C. Membranes were washed 3 x in PBS with 0.1% Tween 20 (PBST) and incubated in goat anti-rabbit HRP conjugated secondary antibody (1/10000 diluted in 5 % milk in PBST) for 2 hours at room temperature. Membranes were washed 3 x in PBST and developed with Advansta western blotting detection kit according to manufacturer's instructions.

### Immunohistochemistry

Mice were euthanised by cervical dislocation. Brains were quickly removed and placed in 4% formaldehyde overnight at 4°C. Fixed brains were washed 3 times in PBS and cryoprotected in 20 % sucrose for 48 hours. Brains were embedded in OCT compound and frozen in a dry ice/ethanol slurry, sectioned to 20µm thickness and processed for immunohistochemistry as below. 20µm mouse brain sections or resected brain sections from the lateral temporal lobe, amygdala, hippocampus and temporal pole from temporal lobe epilepsy patients or autopsy control cases were fixed in methanol for 10min at -20°C. Sections were rehydrated in PBS and blocked in 5% normal goat serum/0.1 % triton x-100 for 30 min at room temperature. Antibody incubations were performed overnight at 4°C. Secondary fluorescent conjugated antibodies were incubated for 1 hour at 37°C. Nuclei were stained with Hoechst for 1 min and slides were coverslipped with Aqua Poly/mount. Images were acquired on a Zeiss LSM 710 confocal microscope.

### Dynamic contrast-enhanced magnetic resonance imaging

All ethical approvals were in place prior to initiation of studies on human subjects. BBB permeability maps were created using the slope of contrast agent concentration in each voxel over time, calculated by a linear fit model as previously described. Thresholds of high permeability was defined by the 95th percentile of all slopes in a previously examined control group (Dong et al., 2015, 2016). Supra-threshold values of individuals were then normalized to pre-season values to determine relative change over the course of play. Additionally, Ktrans maps were also created for each scan. All imaging was performed using a 3T Philips Achieva scanner, and included a T1- weighted anatomical scan (3D gradient echo, TE/TR =3/6.7ms, acquisition matrix 268x266, voxel size: 0.83x0.83x.9mm), T2-weighted imaging (TE/TR =80/3000ms, voxel size: 0.45x0.45x.4mm), FLAIR (TE/TR =125/11000ms, voxel size:0.45x0.45x4mm). For the calculation of pre-contrast longitudinal relaxation time (T10), the variable flip angle (VFA) method was used (3D T1w-FFE, TE/TR = 2.78/5.67ms, acquisition matrix: 240x184, voxel size: 0.68x0.68x5 mm, flip angles: 2,10,16 and 24°). Dynamic contrast enhanced (DCE) sequence was then acquired (Axial, 3D T1w-FFE, TE/TR = 2.78/5.6ms, acquisition matrix: 240x184, voxel size: 0.68x0.68x5mm, flip angle: 6°, Tt = 6.5Sec, temporal repetitions: 70, total scan length: 7.6minutes). An intravenous bolus injection of the contrast agent gadobentate dimeglumine (Gd-BOPTA, Bracco Diagnostics Inc., Milan, Italy) was administered using an automatic injector after the first three DCE repetitions. For the second, older cohort, T1-weighted, T2-weighted and FLAIR imaging parameters were kept the same. For the calculation of pre-contrast longitudinal relaxation time (T10), the variable flip angle (VFA) method was used (3D T1w-FFE, TE/TR = 2.78/5.67ms, acquisition matrix: 208x,204 voxel size: 0.86x0.86x6mm, flip angles:10,15, 20, 25 and 30°). Dynamic contrast enhanced (DCE) sequence was then acquired (Axial, 3D T1w-FFE, TE/TR = 2.78/5.6ms, acquisition matrix: 208x,204 voxel size: 0.86x0.86x6mm, flip angle: 20°, Tt = 22.2Sec, temporal repetitions: 61, total scan length: 22.6 minutes). Intravenous bolus injection of the contrast agent gadobentate dimeglumine was administered using an automatic injector after the first five DCE repetitions.

### Electroencephalography

Mice were anesthetized with isoflurane (isoflurane; 5% induction, 1-2% maintenance) and placed in a mouse-adapted stereotaxic frame. Body temperature was maintained within the normal physiological range with a feedback-controlled heat pad (Harvard Apparatus, Kent, UK; Holliston, MA). After making a midline scalp incision, the bregma was located, and three partial craniectomies were performed for the placement of skull-mounted recording screws (Bilaney Consultants). The electrode assembly was fixed in place with dental cement, and the mouse was placed in a heated chamber for surgery recovery. After full recovery, mice were placed in an open Perspex box, which allowed free movement. The EEG was recorded using a Xltek^{®} brain monitor amplifier (Natus). Mouse EEG data were analyzed and quantified using LabChart 8 software (ADInstruments, Oxford, U.K.). Seizures were defined as high-amplitude (>2 x baseline) high-frequency (>5 Hz) polyspike discharges lasting >5seconds. Status epilepticus was defined as at least 5minutes of continuous seizure activity. From the EEG recordings we calculated the total power and % of total power as previously described by Jimenez-Mateos, E.M., et al., Silencing microRNA-134 produces neuroprotective and prolonged seizure-suppressive effects. Nat Med, 2012. 18(7): p. 1087-94. EEG total power was plotted as percentage of baseline recording (each animal's EEG power post seizure compared to its own baseline EEG).

### Statistical analysis

Charts were generated with Prism 8. Tests used included Student's t-test and one-way ANOVA with Tukey post-test.

### Example 1

### CLDN5 and ICAM2 mRNA expression is restricted to endothelial cells

Figure 1 shows detailed mRNA expression profiles for CLDN5 and ICAM2 in the mouse central nervous system. These expression profiles were generated from single-cell RNA sequencing of cells from the mouse central nervous system. It is important to note that CLDN5 and ICAM2 mRNA expression is restricted to endothelial cells. Therefore, the promoter elements of these genes can be used to drive expression of transgenes specifically in endothelial cells. This is an important consideration for therapeutic delivery of gene therapies specifically to endothelial cells for the treatment of neurological disorders with compromised blood-brain barrier integrity such as mesial temporal lobe epilepsy (mTLE).

### Example 2

### Vectors containing the CLDN5 gene under the control of a CLDN5 or ICAM2 promoter

Figure 2 shows the vector maps which contain the CLDN5 gene which is under the control of either a CLDN5 or ICAM2 promoter. The map highlights a novel approach to use these specific promoter sequences to express a human or mouse CLDN5 cDNA. The vector map shows the requisite features for antibiotic selection of the plasmid and for packaging of the plasmid into adeno-associated viral vectors.

### Example 3

### Using a CLDN5 or ICAM2 promoter to drive expression of claudin-5 protein and mRNA

Figure 3 shows protein and mRNA expression of claudin-5 in human cells that have been transfected with the novel vectors. This shows that using either a CLDN5 or ICAM2 promoter can drive high levels of expression of claudin-5 protein and mRNA compared to cells left un-transfected or transfected with a vector that does not contain claudin-5 cDNA. With this system, 200-300 fold increases in claudin-5 protein and mRNA levels can be achieved. This is vital for a therapeutic strategy of restoring and/or improving claudin-5 levels in neurological disorders with depleted levels of claudin-5.

### Example 4

### Mice with depleted levels of claudin-5 develop seizure activity

Figure 4 shows that mice with depleted levels of claudin-5 develop spontaneous recurrent seizures. With the novel mouse model, levels of claudin-5 in brain endothelial cells can be depleted via the administration of doxycycline to the mouse drinking water. This results in the activation of a short-hairpin RNA (shRNA) which represses claudin-5 mRNA and reduces translation of the claudin-5 protein product. Over time the mice develop spontaneous seizures which we can record by cortical electroencephalography. This reveals that mice with depleted levels of claudin-5 develop seizure activity. This highlights that claudin-5 is an important protein in the development of seizures in mice and that regulating claudin-5 levels could be used to prevent the onset of seizures.

### Example 5

### The pathology of seizures in mice can be reversed

Figure 5 shows that mice with depleted levels of claudin-5 develop astrogliosis in the hippocampus which is a hallmark feature of epilepsy. Importantly, it is shown that by removing doxycycline, which prevents activation of the claudin-5 shRNA, the onset of astrogliosis can be prevented. This crucial step shows that the pathology of seizures in mice can be reversed by controlling the levels of claudin-5 in the model.

### Example 6

### The pattern of claudin-5 in the blood vessels of mTLE patients is severely disrupted

Figure 6 shows dynamic contrast-enhanced magnetic-resonance imaging of the brain in patients with mTLE. These images reveal the extensive blood-brain barrier breakdown that occurs in this condition which we have assessed by the leakage of the small molecule gadolinium. It is also shown that levels of claudin-5 are depleted in the blood vessels in the epileptic focal region that has been surgically removed from the brains of the mTLE patients. This shows that the pattern of claudin-5 in the blood vessels of mTLE patients is severely disrupted. Therefore, the therapeutic strategy of delivering a claudin-5 gene therapy to restore claudin-5 expression levels is a viable option in mTLE patients.

## Claims

1. A tight junction protein for use in treating or preventing epilepsy in a subject, wherein the use comprises administering the tight junction protein to the subject.

2. A tight junction protein for use according to Claim 1, wherein the epilepsy is temporal lobe epilepsy, optionally mesial temporal lobe epilepsy.

3. A tight junction protein for use according to Claim 1 or 2, wherein the epilepsy is drug-resistant epilepsy.

4. A tight junction protein for use according to any one of Claims 1-3, wherein the use is for treating or preventing epileptic seizures, optionally focal epileptic seizures.

5. A tight junction protein for use according to any one of Claims 1-4, wherein the tight junction protein is a claudin protein.

6. A tight junction protein for use according to any one of Claims 1-5, wherein the tight junction protein is a claudin-5 protein.

7. A tight junction protein for use according to any one of Claims 1-6, wherein the use comprises increasing the quantitative or qualitative level of the tight junction protein in an endothelial cell of the brain.

8. A tight junction protein for use according to any one of Claims 1-7, wherein the use comprises administering an agent capable of increasing the quantitative or qualitative level of the tight junction protein in an endothelial cell of the brain to the subject.

9. A tight junction protein for use according to Claim 8, wherein the agent is a nucleic acid encoding the tight junction protein, or is fragment or variant thereof.

10. A tight junction protein for use according to Claim 8, wherein the agent is a vector comprising a first nucleic acid encoding the tight junction protein, or fragment or variant thereof, operatively linked to a second nucleic acid comprising an endothelial-cell-specific promoter, or fragment or variant thereof.

11. A tight junction protein for use according to Claim 10, wherein the first nucleic acid or fragment or variant thereof encodes a claudin-5 protein, and the second nucleic acid comprises a claudin-5 promoter or fragment or variant thereof.

12. A tight junction protein for use according to Claim 10, wherein the first nucleic acid or fragment or variant thereof encodes a claudin-5 protein, and the second nucleic acid comprises an intercellular adhesion molecule 2 (ICAM2) promoter or fragment or variant thereof.

13. A tight junction protein for use according to Claim 10, wherein the first nucleic acid has a nucleic acid sequence according to any one or more of SEQ ID NO:1, SEQ ID NO:2, SEQ ID NO:3, and SEQ ID NO:4; and the second nucleic acid has a nucleic acid sequence according to any one or more of SEQ ID NO:5, and SEQ ID NO:6.

14. A tight junction protein for use according to Claim 10, wherein the vector comprises a first nucleic acid having a nucleic acid sequence according to SEQ ID NO:2, or fragment or variant thereof, operatively linked to a second nucleic acid having a nucleic acid sequence according to SEQ ID NO:6, or fragment or variant thereof.

15. A tight junction protein for use according to Claim 8, wherein the agent is an isolated adeno-associated virus (AAV) comprising a vector as defined in any one of Claims 10-14.


SEQUENCE LISTING

<110>  The Provost, Fellows, Foundation Scholars, & the other
       members of Board, of the College of the Holy & Undiv. Trinity of
       Queen Elizabeth Near Dublin

<120>  A tight junction (zonulae occludentes) protein for use in
       treating or preventing a neurological disorder

<130>  P133865EP00

<160>  6

<170>  PatentIn version 3.5

<210>  1
<211>  912
<212>  DNA
<213>  Homo sapiens

<400>  1
atgacccgcg cacggattgg ctgcttcggg ccggggggcc gggcccgggg gacagaatcc      60

gcccccgaac cttcaaagag ggtacccccc ggcaggagct ggcagaccca ggaggtgcga     120

cagacccgcg gggcaaacgg actggggcca agagccggga gcgcgggcgc aaaggcacca     180

gggcccgccc agggcgccgc gcagcacggc cttgggggtt ctgcgggcct tcgggtgcgc      240

gtctcgcctc tagccatggg gtccgcagcg ttggagatcc tgggcctggt gctgtgcctg     300

gtgggctggg ggggtctgat cctggcgtgc gggctgccca tgtggcaggt gaccgccttc     360

ctggaccaca acatcgtgac ggcgcagacc acctggaagg ggctgtggat gtcgtgcgtg     420

gtgcagagca ccgggcacat gcagtgcaaa gtgtacgact cggtgctggc tctgagcacc     480

gaggtgcagg cggcgcgggc gctcaccgtg agcgccgtgc tgctggcgtt cgttgcgctg     540

ttcgtgaccc tggcgggcgc gcagtgcacc acctgcgtgg ccccgggccc ggccaaggcg     600

cgtgtggccc tcacgggagg cgtgctctac ctgttttgcg ggctgctggc gctcgtgcca     660

ctctgctggt tcgccaacat tgtcgtccgc gagttttacg acccgtctgt gcccgtgtcg     720

cagaagtacg agctgggcgc agcgctgtac atcggctggg cggccaccgc gctgctcatg     780

gtaggcggct gcctcttgtg ctgcggcgcc tgggtctgca ccggccgtcc cgacctcagc     840

ttccccgtga agtactcagc gccgcggcgg cccacggcca ccggcgacta cgacaagaag     900

aactacgtct ga                                                         912


<210>  2
<211>  912
<212>  DNA
<213>  Homo sapiens

<400>  2
atgactaggg cccggattgg atgcttcggt cccggcggac gcgcgcgcgg aaccgagtca      60

gcccccgaac cgagcaaacg cgtgccaccc ggaagatcat ggcagaccca agaagtcaga     120

cagacccggg gcgccaacgg gctgggtcct cgggccggat ccgcgggggc caaggcacct 180

gggccagctc agggtgccgc acagcacggc ctgggcggaa gcgcagggtt gagggtccgg 240

gtgtcccctc tggccatggg atcggccgcc ctcgagattt tgggcctggt gctgtgcctg 300

gtcggatggg gcggactgat cctggcctgc ggactcccga tgtggcaagt caccgccttt 360

ctcgatcata acatcgtgac ggcccagacc acctggaagg gcctgtggat gtcgtgcgtg 420

gtgcagtcca ctggccacat gcagtgcaag gtctacgact ccgtgctggc gctgtccact 480

gaggtgcagg ccgcccgggc cctcactgtg tccgccgtgc ttctcgcttt cgtggcgctg 540

ttcgtgaccc tggccggtgc tcagtgcact acctgtgtgg cgccgggccc cgccaaggcc 600

cgcgtggccc tgactggggg agtgctgtac ctcttctgtg gcctccttgc cctcgtgccg 660

ctgtgctggt tcgcgaatat cgtggtccgg gaattctatg acccttcggt gccggtgtcc 720

caaaaatacg aacttggtgc tgcactgtac atcggatggg ccgcaacagc gctgctgatg 780

gtcggcggct gcctgctgtg ctgcggagcc tgggtctgta ccggacgccc cgacttgagc 840

ttccccgtga agtacagcgc ccctagacgg ccgaccgcta ccggggatta cgacaagaag 900

aactacgtgt ga 912

<210> 3
<211> 657
<212> DNA
<213> Mus musculus

<400> 3
atggggtctg cagcgttgga aattctgggt ctggtgctgt gtctggtagg atgggtgggc 60

ttgatcctgg cgtgtgggct gcccatgtgg caggtgactg ccttcctgga ccacaacatc 120

gtgacggcgc agacgacttg gaaggggctg tggatgtcgt gcgtggtgca gagtaccggg 180

cacatgcagt gcaaggtgta tgaatctgtg ctggcgctga gtgcggaggt gcaggcagct 240

cgggcactca ccgtgggcgc tgtgctgctg gcgctggtgg cactctttgt taccttgacc 300

ggcgctcagt gcaccacctg cgtggccccg ggcccagtta aggcacgggt agcactcacg 360

ggaggagcgc tttacgcggt gtgcgggctg ctggcactcg tgccgctctg ctggttcgcc 420

aacatcgttg tccgcgagtt ctatgatccg acggtgccgg tgtcacagaa gtacgagctg 480

ggcgcggcgc tgtacatcgg ctgggcggcc tccgcactgc tcatgtgcgg tggcggcctc 540

gtgtgttgcg gcgcctgggt ctgcaccggg cgccctgagt tcagcttccc ggtcaagtac 600

tctgcgccgc ggcggcccac ggccaatggc gattacgaca agaagaacta tgtctaa 657

<210> 4
<211> 657
<212> DNA
<213> Mus musculus

<400>  4
atgggaagcg ccgcattgga aatcctcggg ctggtgctgt gcctcgtggg atgggtcgga      60

ctgattctgg cttgcggact gccgatgtgg caagtcaccg ccttcctgga ccacaacatc     120

gtgactgcgc agaccacttg gaagggtctg tggatgtcgt gtgtggtcca gtccaccggc     180

catatgcagt gcaaagtcta cgagtccgtg ctcgccttgt ccgccgaggt gcaagctgcg     240

agagccctga ccgtgggggc cgtgctgctg gccctggtgg cgcttttcgt gacactgacg     300

ggtgcccagt gtactacctg tgtggccccg ggccccgtga aggcccgggt ggccctcact     360

ggcggagccc tgtacgccgt gtgcgggctg ctggcgctgg tgcccctgtg ctggttcgcc     420

aacatcgtcg tgcgcgagtt ctacgatcct accgtgcccg tcagccagaa atacgaactt     480

ggagccgctc tgtatattgg ctgggctgca tcggccctcc ttatgtgcgg tggcggcctc     540

gtgtgctgcg gggcatgggt ctgcaccgga cgcccggaat tctcctttcc tgtgaagtac     600

tcagccccac ggaggcctac cgcgaacgga gactacgaca agaagaatta cgtgtga        657


<210>  5
<211>  1169
<212>  DNA
<213>  Homo sapiens

<400>  5
aacaaaggga gcagggagga gtgttcactg gaaagaggct ggcgccctag tttggttgtg      60

ggctgtgggg cctggtgccc ccacctgagc ctcagggacc cagtgtgtct aacccagtgg     120

acctttcaag aaatggctgg gccattgtgc agaagaatgc ccggaaatcc cgcggctccc     180

tcctccaccg aggatggggg ctcttgctcc tggccaggaa actccaagtt ggcttccgga     240

gggtggcctg gggggctggg tggcaaagca ccacaactat ttgtaggatg gcaaggcata     300

gcctttctat gtcccatcgt gtctctgctg tcccaggatt ccagtatgct gtgttaggat     360

cacagcctat ctgctggaga tgactggacg tcggagaacc ctcaggggtt gcgcagcatc     420

ccagaggaga gcacaggccc ttgcagtgtt cactttgtgt cagaccagtg gttccaggga     480

aggagtgaga actgtaggcg gtggttgggt aggatcatga atgaggttat aattaagggc     540

aagcccaaca caggcttgcc gagcatctcc gcttggtgtg ccaaggaaaa aacggcgggc     600

gtagaggttt gtttcacctg tgcacggggg ctcagagaac ctccaggcca agcagtctgc     660

agagaggagt tggaatgatc cgggatgtag caggtggagc tggtgagtgt aggttagggg     720

tcatgctgct aacagtggag acaggggcgc acagatctcc tgtgtacctg ccaagatcag     780

cccctactag gacagaaact ggtagggact cagactgcgg ttctgcaggg tgcggttcgg     840

gtgagcagga ctagtcctgc tcgcccgtgg tttcagtaac tgtagctgtc agggggtccg      900

cgattggctg tgggccgaag gtctgctcat ggattggttg agctaactcg gggggctgcg     960

ccctgggggc agagtccgcc cccgagactt caaataggac aacaggttac ttcagaaccc 1020

agttggtgta gttaaaacct cctcttctgc tccaggactg gaggctccag agcagaggca 1080

ccagaatcaa ttcccagctc ccagcctaag cagcgcagag agcacccgga ggccccaagg 1140

gccgtcgggt gagcattcag tctttagcc 1169

<210> 6
<211> 367
<212> DNA
<213> Homo sapiens

<400> 6
atgggatttg gggttcccca gatctggggc ttgtaggcct gactctcccc tgtgcacacg 60

tctcatacac gcatgcgtgc acccattgcc tgccccgccc cttgcacagg gagtcagcag 120

ggaggactgg gttatgccct gcttatcagc agcttcccag cttcctctgc ctggattctt 180

agaggcctgg ggtcctagaa cgagctggtg cacgtggctt cccaaagatc tctcagataa 240

tgagaggaaa tgcagtcatc agtttgcaga aggctaggga ttctgggcca tagctcagac 300

ctgcgcccac catctccctc caggcagccc ttggctggtc cctgcgagcc cgtggagact 360

gccagag 367